Europäisches Patentamt

European Patent Office

Office européen des brevets

(11) Veröffentlichungsnummer: **0 316 554 B1**

# EUROPÄISCHE PATENTSCHRIFT

(45) Veröffentlichungstag der Patentschrift: **30.12.92**

(21) Anmeldenummer: **88116292.9**

(22) Anmeldetag: **01.10.88**

(51) Int. Cl.⁵: **A61B** 1/00, G02B 23/24

(54) **Steckvorrichtung für Endoskope.**

(30) Priorität: **16.11.87 DE 3738633**

(43) Veröffentlichungstag der Anmeldung:
**24.05.89 Patentblatt 89/21**

(45) Bekanntmachung des Hinweises auf die
Patenterteilung:
**30.12.92 Patentblatt 92/53**

(84) Benannte Vertragsstaaten:
**BE DE FR GB NL**

(56) Entgegenhaltungen:
**EP-A- 0 037 013**
**DE-A- 3 624 442**
**US-A- 4 422 702**
**US-A- 4 617 915**
**US-A- 4 618 195**

(73) Patentinhaber: **Richard Wolf GmbH**
**Pforzheimer Strasse 32**
**W-7134 Knittlingen(DE)**

(72) Erfinder: **Ams, Felix**
**Gründle Strasse 5**
**W-7539 Kämpfelbach(DE)**

(74) Vertreter: **Wilcken, Thomas, Dipl.-Ing. et al**
**Musterbahn 1**
**W-2400 Lübeck(DE)**

## Beschreibung

Die Erfindung geht aus von einem Steckverbinder gemäß dem Oberbegriff des Patentanspruchs 1. Ein solcher Steckverbinder ist in der DE-A-36 24 442 beschrieben.

Zum Anschluß eines flexiblen Endoskops an Versorgungseinheiten wie Lichtquelle, Saug- und Spülpumpe weist das Endoskop entsprechend dem vorbekannten Stand der Technik eine an seinem Steuergehäuse festgelegte Versorgungsleitungsanordnung auf, deren distales Ende in zwei voneinander getrennte Versorgungsleitungen mit entsprechenden Steckverbindungen aufgeteilt ist. Diese Aufteilung ist deshalb erforderlich, weil es sich bei der benötigten Lichtquelle und der Saug- und Spülpumpe in der Regel um jeweils zwei getrennte Versorgungseinheiten handelt, die somit kein gemeinsames Anschlußfeld aufweisen; auch dann nicht, wenn diese Einheiten beispielsweise gemeinsam in einem Gerätewagen oder dergleichen angeordnet oder in diesem als Geräteeinschub eingesetzt sind.

Weil andererseits auch kombinierte Einheiten, bestehend aus einer Lichtquelle und einer Saug- und Spülpumpe, in einem gemeinsamen Gehäuse mit kombiniertem Anschlußfeld und darauf angeordneten Anschlußbuchsen in Gebrauch sind, wie es zum Beispiel auch aus der DE-A-36 24 442 hervorgeht, können die beiden einzelnen Stecker der flexiblen Endoskope der vorgenannten Art aus Abstandsgründen im allgemeinen nicht mit kombinierten Versorgungseinheiten benutzt werden. Gemäß der vorgenannten Druckschrift sind die Steckanschlußgruppen des Steckverbinders der biegsamen Versorgungsleitungsanordnung des Endoskopes im wesentlichen in gleichbleibender radialer Beziehung zueinander angeordnet. Nur aus Gründen der Vermeidung von engen Fertigungstoleranzen sind die aufnehmenden Gegensteckanschlußgruppen der den Steckverbinder aufnehmenden, kombinierten Steckbuchse der kombinierten Versorgungseinheit innerhalb gewisser Grenzen in radialer Richtung nachgiebig, um ein sicheres Zusammenstecken der Steckanschlüsse zu gewährleisten.

Die Aufgabe der Verbindung besteht in der Verbesserung eines Steckverbinders der einleitend angeführten Art dahingehend, daß die Verbindung eines flexiblen Endoskopes sowohl mit getrennten Versorgungseinheiten als auch mit einer kombinierten Versorgungseinheit möglich ist.

Diese Aufgabe wird, ausgehend von dem einleitend angeführten Steckverbinder, erfindungsgemäß dadurch gelöst, daß am Gehäuse des Steckverbinders eine Aufnahme zur lösbaren Anbringung eines Steckers des Lichtleitkabels an dem Gehäuse vorgesehen ist.

Die durch diese Lösung erzielbaren Vorteile liegen insbesondere in der leichten und schnellen Anpaßbarkeit des distalen Steckverbinders des flexiblen Endoskopes an die jeweils vorhandene Ausführungsform der Versorgungseinheit bzw. Versorgungseinheiten. Soll eine kombinierte Versorgungseinheit angewendet werden, so wird das normalerweise getrennte Lichtleitkabel an seinem Stecker an dem Gehäuse des übrigen Steckverbinders angeklippt, so daß eine passende Lage des Steckers des Lichtleitkabels gegeben ist. Hierbei wird bei einfacher, sicherer und schneller Handhabbarkeit des Steckverbinders eine übersichtliche Anordnung, Aufbewahrung und Zusammenfassung der Versorgungsleitungen erreicht. Die Konstruktion für die Aufnahme des Steckers des Lichtleitkabels ist sehr einfach und mit nur geringen Kosten durchführbar.

Die vorteilhaften Eigenschaften der Erfindung kommen besonders dann zum Tragen, wenn die erfindungsgemäße Aufnahme aus elastisch deformiebaren Klemmbacken besteht, die einen Einsteckschlitz für den Stecker des Lichtleitkabels bilden und diesen umfassen, und wenn der Stecker durch eine formschlüssig in diesen eingreifende Halterung gegen axiales Verschieben gesichert ist.

Bei einer weiteren bevorzugten Ausführungsform kann vorgesehen sein, daß die Aufnahme zusammen mit der Halterung für die axiale Fixierung des Lichtleitkabels lösbar mit dem Gehäuse verbunden ist. Dadurch ist die Möglichkeit gegeben, die Aufnahme für den Stecker des Lichtleitkabels im Falle eines separaten Anschlusses desselben von dem Gehäuse zu entfernen, um eine mögliche Behinderung durch die Aufnahme beim Steckvorgang zu vermeiden.

Schließlich kann zur Verbesserung der Anschlußsicherheit und im Sinne einer kompakten Ausführung des Steckverbinders vorgesehen sein, daß die Anschlüsse für die elektrische Versorgung in einer Steckerleiste zusammengefaßt sind, die wie die übrigen Anschlüsse im stirnseitigen Ende des Steckverbinders angeordnet sind. Hierbei kann eine eventuell zusätzlich erforderliche Steuereinheit wahlweise in der Lichtquelleneinheit oder in der Saug- und Spülpumpe angeordnet sein.

Die Erfindung ist nachstehend anhand eines in der anliegenden Zeichnung dargestellten Ausführungsbeispieles näher erläutert. Es zeigen:

Figur 1     einen erfindungsgemäßen Steckverbinder mit angekuppeltem Lichtleitkabel,

Figur 2     der Steckverbinder nach Figur 1 in Seitenansicht,

Figur 3     eine Aufnahme zum Festlegen des Lichtleitkabels am übrigen Steckverbinder,

Figur 4     eine Halterung zum Festlegen des Lichtleitkabels in axialer Richtung.

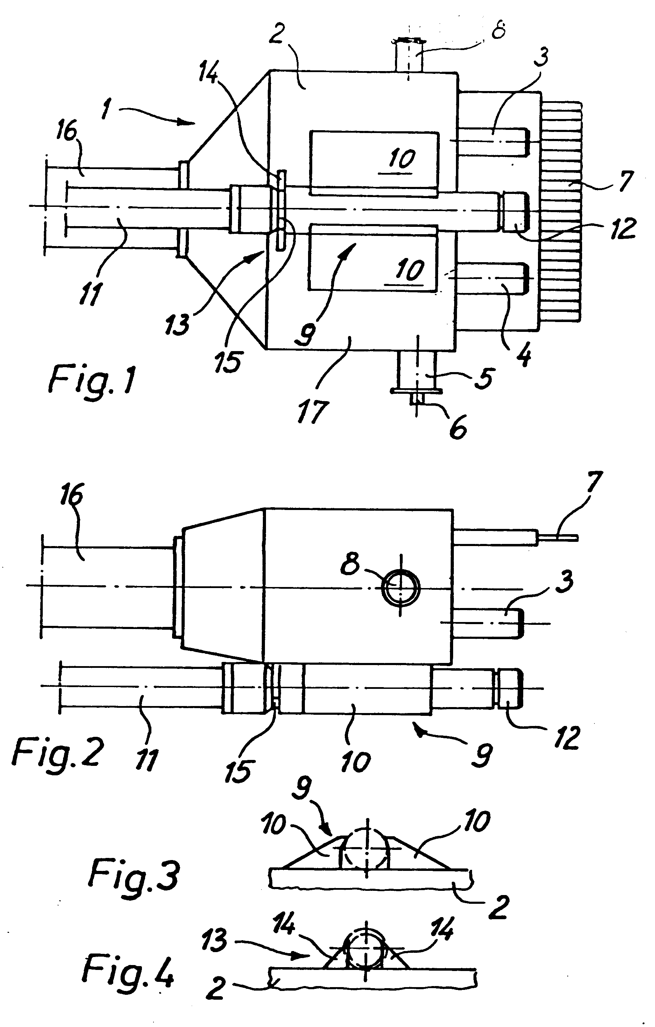

Wie aus der Figur 1 ersichtlich ist, weist das Gehäuse 2 der Steckvorrichtung 1 an seinem stirnseitigen Ende eine erste rohrförmige Steckverbindung 3 zum Zuführen eines Körperhöhlengases auf. Weiterhin ist eine zweite rohrförmige Steckverbindung 4 zum Zuführen von Druckluft vorgesehen, die über den inneren Kanal 6 eines Anschlusses 5 in ein damit zu verbindendes Flüssigkeitsaufnahmegefäß geleitet wird, um durch den dadurch in diesem Gefäß erzeugten Überdruck die Flüssigkeit durch einen koaxial um den Kanal 6 gebildeten Ringkanal in die Körperhöhle einzuleiten. Zur Übertragung elektrischer Signale oder Steuerbefehlen und dgl. ist im Abstand zu den beiden Steckverbindungen 3 und 4 eine Steckerleiste 7 mit einer Vielzahl von Steckkontakten angeordnet. Über einen seitlich angeordneten Anschluß 8 kann ein weiteres flaschenförmiges Auffanggefäß zur Aufnahme von aus der Körperhöhle abgesaugtem Sekret angeschlossen werden. Das Gehäuse 2 ist weiter an einer Seitenfläche mit einer Aufnahme 9 versehen. Sie besteht aus zwei aus einem geeigneten elastischen Werkstoff geformten Klemmbacken 10, welche einander unter Belassung eines sich parallel zu der Achse des Gehäuses 2 erstreckenden Einsteckschlitzes gegenüberstehen und im Bereich ihrer oberen Kanten zugeneigt sind. Zum lösbaren Festlegen eines Steckers 12 eines Lichtleitkabels 11 ist es lediglich erforderlich, diesen in Richtung des Einsteckschlitzes auf die beiden parallel zueinander angeordneten Klemmbacken 10 zu legen und sodann einen leichten Druck auf diesen auszuüben. Durch den dadurch auf die oberen Kanten der beiden Klemmbacken 10 wirkenden Druck werden diese elastisch auseinandergespreizt, so daß der Stecker 12 des Kabels 11 zwischen Klemmbacken 10 zu liegen kommt und durch deren Spannkraft lösbar festgelegt wird. Um nun beim gemeinsamen Einführen der Steckvorrichtung 1 und des Steckers 12 des Kabels 11 in die entsprechenden Buchsen des Versorgungsgerätes ein axiales Verschieben des Steckers 12 zu vermeiden, ist eine Halterung 13 mit Vorsprüngen 14 vorgesehen, die in eine Ringausnehmung 15 am proximalen Ende des Steckers 12 eingreifen und diesen zusätzlich in axialer Richtung festlegen. Durch die beiden Klemmbacken 10 einerseits und die beiden Vorsprünge 14 andererseits wird eine gute, sichere und schnelle Verbindung zwischen der Vorrichtung 1 und dem Stecker 12 des Lichtleitkabels 11 geschaffen.

Wie in Figur 1 angedeutet ist, kann die Aufnahme 9 gemeinsam mit der Halterung 13 in einem Bauelement 17 vereinigt sein, welches an dem Gehäuse 2 lösbar angebracht sein kann. Eine solche Verbindung kann beispielsweise über eine Schwalbenschwanzführung erfolgen, die gestattet, das Bauelement 17 von dem die Anschlüsse 3 und 4 tragenden stirnseitigen Ende des Gehäuses 2 aus in dieses einzuschieben.

Im Zusammenhang mit der Erfindung ist der Vollständigkeit halber noch festzuhalten, daß sich an den Stecker 12 das Lichtleit kabel 11 anschließt und daß die Versorgungsleitung 16 alle Schlauchverbindungen und elektrische Leitungen aufnimmt, die zwischen der Steckvorrichtung 1 und dem Endoskop verlaufen.

## Patentansprüche

1. Steckverbinder eines mit Versorgungsleitungen einschließlich eines Lichtleitkabels versehenen Endoskopes zum Anschluß an Versorgungseinheiten, wie Lichtprojektoren, Pumpen und elektrische Generatoren, bestehend aus einem Gehäuse (2) mit Anschlüssen (3,4,7), die mit entsprechenden Anschlüssen der jeweiligen Versorgungseinheit bzw. Versorgungseinheiten verbindbar sind, dadurch gekennzeichnet, daß am Gehäuse (2) des Steckverbinders (1) eine Aufnahme (9) zur lösbaren Anbringung eines Steckers (12) des Lichtleitkabels (11) an dem Gehäuse (2) vorgesehen ist.

2. Steckverbinder nach Anspruch 1, dadurch gekennzeichnet, daß die Aufnahme (9) aus elastisch deformierbaren Klemmbacken (10) besteht, die einen Einsteckschlitz für den Stecker (12) des Lichtleitkabels(11) bilden und diesen umfassen, und daß der Stecker (12) durch eine formschlüssig in diesen eingreifende Halterung (13) gegen axiales Verschieben gesichert ist.

3. Steckverbinder nach einem der Ansprüche 1 und 2, dadurch gekennzeichnet, daß die Aufnahme (9) zusammen mit der Halterung (13) lösbar mit dem Gehäuse (2) verbunden ist.

4. Steckverbinder nach einem der Ansprüche 1 bis 3, dadurch gekennzeichnet, daß die Anschlüsse für die elektrische Versorgung in einer Steckerleiste (7) zusammengefaßt sind, die wie die übrigen Anschlüsse (3 u. 4) am stirnseitigen Ende des Steckverbinders angeordnet sind.

## Claims

1. A plug connector of an endoscope, provided with supply lines including a photoconducting cable, for connection to supply units, such as light projectors, pumps and electric generators, consisting of a housing (2) with connections (3, 4, 7), which are able to be connected with corresponding connections of the respective supply unit or respectively supply units, characterised in that there is provided on the housing (2) of the plug connector (1) a mounting (9) for the detachable arrangement of a plug (12) of the photoconducting cable (11) on the housing (2).

2. A plug connector according to Claim 1, characterised In that the mounting (9) consists of elastically deformable clamping jaws (10), which form an insertion slot for the plug (12) of the photoconducting cable (11) and surround the latter, and that the plug (12) is secured with respect to axial displacement by means of a holder (13) engaging therein in a form-locking manner.

3. A plug connector according to one of Claims 1 and 2, characterised in that the mounting (9) together with the holder (13) is detachably connected with the housing (2).

4. A plug connector according to one of Claims 1 to 3, characterised in that the connections for the electrical supply are combined in a plug board (7), which, like the remaining connections (3 and 4) are arranged on the front end of the plug connector.

**Revendications**

1. Connecteur de liaison d'un endoscope pourvu de conduites d'alimentation, y compris un câble formant conducteur de lumière, destiné au raccordement à des unités d'alimentation, telles que des projecteurs de lumière, des pompes et des générateurs électriques, et constitué par un boîtier (2) comportant des raccords de branchement (3, 4, 7) pouvant être reliés à des raccords de branchement correspondants de l'unité d'alimentation ou des unités d'alimentation considérées, caractérisé en ce que sur le boîtier (2) du connecteur de liaison (1), est prévu un logement de réception (9) destiné à fixer de manière amovible sur le boîtier (2), une fiche (12) du câble formant conducteur de lumière (11).

2. Connecteur de liaison selon la revendication 1, caractérisé en ce que le logement de réception (9) est constitué par des mors de serrage (10) déformables de manière élastique, qui forment une fente d'introduction pour la fiche (12) du câble formant conducteur de lumière (11) en l'enserrant, et en ce que la fiche (12) est bloquée à l'encontre d'un coulissement axial, par un support (13) s'engageant par complémentarité de forme dans cette fiche.

3. Connecteur de liaison selon l'une des revendications 1 et 2, caractérisé en ce que le logement de réception (9), y compris le support (13), est relié de manière amovible au boîtier (2).

4. Connecteur de liaison selon l'une des revendications 1 à 3, caractérisé en ce que les raccords pour l'alimentation électrique sont regroupés dans une barrette de connexion (7), et sont disposés, comme les autres raccords (3 et 4), sur l'extrémité frontale du connecteur de liaison.

Fig.1

Fig.2

Fig.3

Fig.4